# EUROPEAN PATENT SPECIFICATION

(11) **EP 2 761 684 B1**
(45) Date of publication and mention of the grant of the patent: **31.10.2018**
(21) Application number: 12766430.8
(22) Date of filing: 24.09.2012
(51) Int. Cl.: H01L 51/00, C07C 211/61, C09K 11/06

(54) **SPIROBIFLUORENE COMPOUNDS FOR LIGHT EMITTING DEVICES**
SPIROBIFLUORENVERBINDUNGEN FÜR LICHTEMITTIERENDE VORRICHTUNGEN
COMPOSÉS DE SPIROBIFLUORÈNE POUR DISPOSITIFS ÉLECTROLUMINESCENTS

(30) Priority: 28.09.2011 EP 11007867
(43) Date of publication of application: 06.08.2014
(73) Proprietor: Sumitomo Chemical Co., Ltd, Chuo-ku, Tokyo 104-8260 (JP)
(72) Inventor: BASCOUR, Dominique, 1390 Grez-doiceau (BE); MAUNOURY, Jonathan, 1030 Brussels (BE); ORSELLI, Enrico, 50677 Köln (DE)
(74) Representative: Dr. Langfinger & Partner
(86) International application number: PCT/EP2012/068805
(87) International publication number: WO 2013/045408

(56) References cited:
- WO-A2-2004/058912
- JP-A- 2005 085 599
- US-A1- 2002 182 439

## Description

The present invention relates to compounds based on spirobifluorene and light emitting devices comprising said compounds.

Various organic light emitting devices have been under active study and development, particularly those based on electroluminescence (EL) from small organic materials. For such organic devices, the ability to form morphologically stable amorphous films is a key requirement for the development of small materials for organic light emitting diodes (OLEDs). That is because when a small molecule compound is used in the organic light-emitting layer, crystallization usually occurs if the molecule of the compound is too small and its structure is too symmetrical. Therefore, when applied in an organic light-emission layer, the small molecule compound is vulnerable to morphological change such as crystallization, and once the crystal is formed, it yields negative impacts upon the light-emitting nature and service life of the OLED.

Thermal stress during device operation can lead to such phase transitions from the amorphous state to the thermodynamically stable polycrystalline state leading to dramatic degradation of the device. As a result it is crucial to design materials featuring high glass transition temperature (Tg >150°C) in order to stabilize the amorphous state. For improving the stability of devices in order to increase operational lifetime, several host materials have been reported. Especially, designing materials having a spiro linkage has been a very successful strategy to obtain OLEDs materials with enhanced morphological stability while keeping their electro-optical functionality.

US2006/0141287 discloses light-emitting layers which include a solid organic material containing a mixture of at least two components. The first host component is an organic compound capable of transporting electrical charges and also forms an aggregate. The second component of the mixture is an organic compound capable of transporting electrical charges and, upon mixing with the first host component, is capable of forming a continuous and substantially pin-hole-free layer. In the reference, as the second component, various compounds such as substituted fluorene derivatives, and spirobifluorene derivatives, etc. are used.

Spirobifluorene, as used herein denotes a structural element of formula (1) and is referred to as SBF hereinafter, whereas Open SBF denotes a system of formula (2) below.

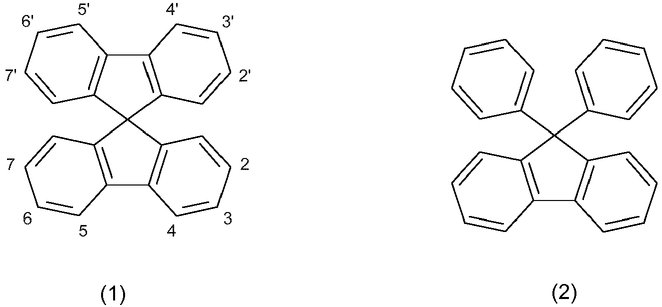

The substitution of the SBF ring system in "para-position" (i.e. the 2, 7, 2' or 7' position of the SBF) by a heteroatom, which carries one or two substituents has been described in the prior art.

2,7'-bis-N.N'-diphenylamino-SBF and various derivatives as well as various derivatives of 2-N,N'-diphenylamino-SBF have been described by Salbeck et al., Chem.Rev. 2007, 107, 1011-1065.

2,2'-disubstituted SBF derivatives with diarylamino-substitutents are also disclosed in US 7,714,145.

Spirobifluorenes with substituted diphenylamino-substituents in 4 and or 4'-position are disclosed in WO 2011/006574. Such compounds may be referred to as orthosubstituted relative to the direct bond linking the phenyl rings of the SBF unit.

European Patent Application 2 312 667 discloses compositions for organic electroluminescence elements comprising at least two different materials fulfilling a certain mathematical equation related to the solubility of the materials. Among an extended list of suitable materials having as a common structural feature substituted diphenylamino groups, 3,6-Bis-N,N'-di(4-tert.butylphenyl)amino-spirobifluorene as well as the respective Open SBF derivative are mentioned.

JP 2005/085599 discloses organic electroluminescent elements including a luminescent layer between a pair of electrodes, which luminescent layer comprises a compound of general formula (1)

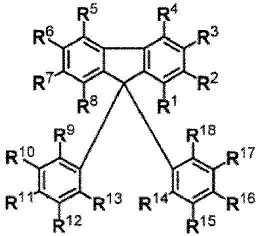

In which at least one of R⁹ to R¹³ and R¹⁴ to R¹⁸ are each a substituted or unsubstituted amino group.

US 2002/182439 describes Open SBF compounds having a symmetrical substitution pattern.

WO 2004/059812 relates to electroluminescent materials and discloses SBF compounds having a symmetrical substitution pattern.

None of the above-disclosed materials meets all the requirements necessary for OLED application, particularly suitable energy level for high phosphorescent efficiency (high triplet energy), high morphological stability, while maintaining other electro-optic and processing properties under operational conditions of the device, such as emission colour, dimensional stability, etc, in a fully satisfactory manner. Thus, there has been a need to develop new host materials, which are capable of satisfying all of the requirements indicated above.

Surprisingly, it has been found that spirobifluorene compounds as defined in claim 1 possess a property spectrum which makes them particularly suitable for use in organic electroluminescent devices.

Preferred compounds in accordance with the instant invention are described in the dependent claims and the detailed specification hereinafter.

The compounds of the present invention are characterized by formula 3 below 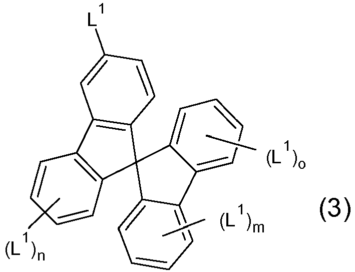
wherein n, m and o are 0 ,
each of the phenyl rings may carry no ligands other than L¹ or may be substituted by ligands other than L¹,
L¹, is represented by the following formula 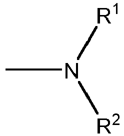 wherein R¹ and R² independently from each other are unsubstituted or substituted phenyl or naphthyl

The phenyl or napththyl groups, may be unsubstituted or substituted by substituents selected from the group consisting of halogen, alkyl, alkoxy, amino, cyano, alkenyl, alkynyl, arylalkyl, aryl and heteroaryl groups or the aryl group may be part of an annealed ring system.

Preferred compounds are compounds of general formula 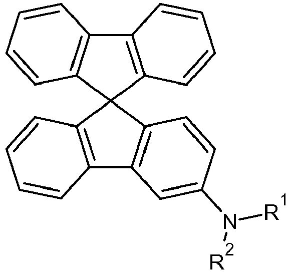 the compound 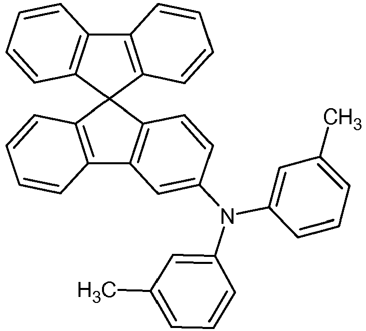 being especially preferred.

The SBF ring system may or may not comprise further substituents in addition to substituents L¹. If present, such additional substituents, which may be the same or different in each position they occur, are generally selected from of halogen, alkyl, alkoxy, amino, cyano, alkenyl, alkynyl, arylalkyl, aryl and heteroaryl groups.

The compounds in accordance with the present invention may be synthesized by any known and suitable method.

Generally, the compounds of the present invention may be prepared by the following general reaction schemes, which show an exemplary way for compounds carrying one or two ligands L¹ 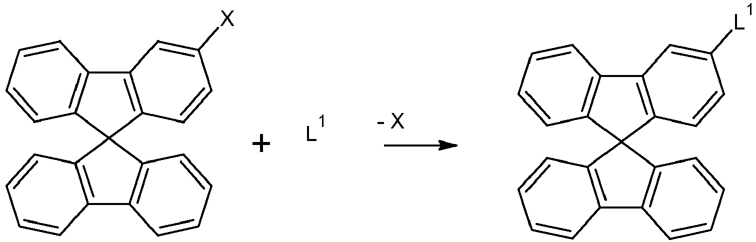 wherein X is a leaving group selected from known leaving groups for such reactions such as halogen, OH, OR, SR, OCN, SCN or CN, especially preferably halogen, in particular chlorine or bromine.

The skilled person will select the suitable reactants and reaction conditions based on the individual needs of a specific synthesis.

The starting materials for such synthesis with at least one leaving group in a meta-position of the SBF ring system may be synthesized in accordance with various process routes which the skilled person will select in accordance with the specific needs. Generally, such compounds are not easily accessible through introduction of the substituents directly into a SBF core as these routes generally yield the para-substituted products preferably due to their higher reactivity. Accordingly, the substituents X have to be introduced through suitable precursor substances e.g. fluorene derivatives, benzophenone derivatives or biphenyl derivatives, to mention only three examples, which are thereafter reacted to yield the SBF structure.

Thus respective compounds may for example be obtained from substituted fluorenone derivatives of formula 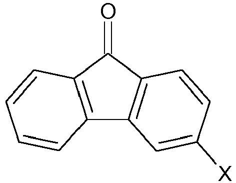 with suitable biphenyl compounds.

Another possibility for the synthesis of the compounds of the present invention is the reaction of fluorenones with suitable substituted biphenyl compounds in accordance with the general reaction scheme 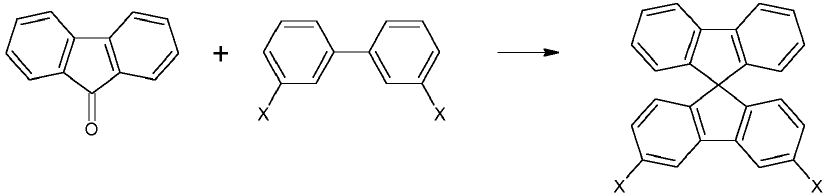 which is described in more detail in JP 2006/089585 for X= OH and which may be adopted for other substituents X.

Another embodiment of the present invention is directed to the use of the compounds of the present invention in an organic light emitting device, especially an organic light emitting diode (OLED).

The compounds in accordance with the present invention may particularly be used as host in an emissive layer in an organic light emitting diode, it is also possible, however, to advantageously use the compounds in accordance with the present invention in other layers of organic light emitting diodes or organic

The present invention is also directed to an organic light emitting device (OLED) comprising an emissive layer (EML), said emissive layer comprising the compounds of the present invention as host material, together with an emitting material.

An OLED generally comprises :
a substrate, for example (but not limited to) glass, plastic, metal;
an anode, generally transparent anode, such as an indium-tin oxide (ITO) anode;
a hole injection layer (HIL) for example (but not limited to) PEDOT/PSS;
a hole transporting layer (HTL);
an emissive layer (EML);
an electron transporting layer (ETL);
an electron injection layer (EIL) such as LiF, Cs₂CO₃
a cathode, generally a metallic cathode, such as an Al layer.

For a hole conducting emissive layer, one may have a hole blocking layer (HBL) that can also act as an exciton blocking layer between the emissive layer and the electron transporting layer. For an electron conducting emissive layer, one may have an electron blocking layer (EBL) that can also act as an exciton blocking layer between the emissive layer and the hole transporting layer. The emissive layer may be equal to the hole transporting layer (in which case the exciton blocking layer is near or at the anode) or to the electron transporting layer (in which case the exciton blocking layer is near or at the cathode).

The compounds of the present invention may be used preferably used as hosts in an emissive layer.

Optionally, the emissive layer may also contain a polarization molecule, present as a dopant in said host material and having a dipole moment that generally affects the wavelength of light emitted.

A layer formed of an electron transporting material is advantageously used to transport electrons into the emissive layer comprising the light emitting material and the (optional) host material. The electron transporting material may be an electron-transporting matrix selected from the group of metal quinoxolates (e.g. Alq₃, Liq), oxadiazoles, triazoles and ketones (e.g. Spirobifluorene ketones SBFK). Examples of electron transporting materials are tris-(8-hydroxyquinoline)aluminum of formula ["Alq₃"] and spirobifluoreneketone SBFK:

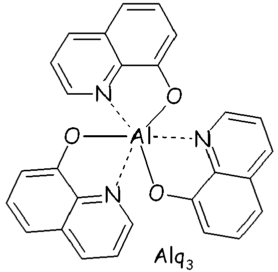

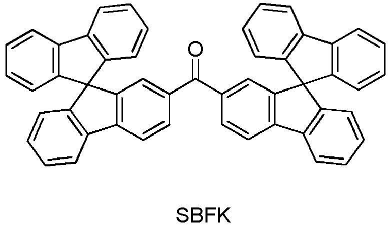

A layer formed of a hole transporting material is advantageously used to transport holes into the emissive layer comprising the light emitting material as above described and the (optional) host material. An example of a hole transporting material is 4,4'-bis[N-(1-naphthyl)-N-phenylamino]biphenyl ["α-NPD"].

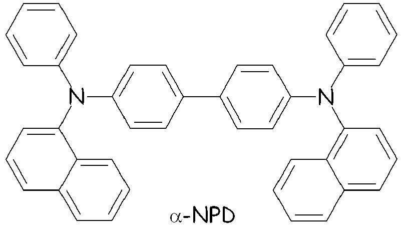

The use of an exciton blocking layer ("barrier layer") to confine excitons within the luminescent layer ("luminescent zone") is greatly preferred. For a hole-transporting host, the blocking layer may be placed between the emissive layer and the electron transport layer. An example of a material for such a barrier layer is 2,9-dimethyl-4,7-diphenyl-1,10-phenanthroline (also called bathocuproine or "BCP"), which has the formula

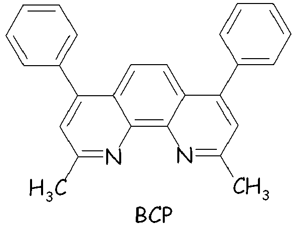

### BCP

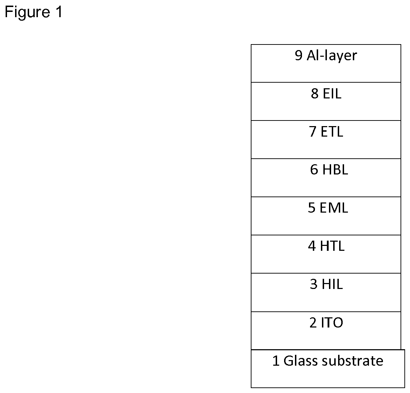

The OLED has preferably a multilayer structure, as depicted in Figure 1, wherein **1** is a glass substrate, **2** is an ITO layer, **3** is a HIL layer comprising PEDOT/PSS, **4** is a HTL layer comprising α-NPD, **5** is an EML comprising mCBP as host material and the light emitting material or mixture of these materials as above defined as dopant in an amount of about 15 % wt with respect to the total weight of host plus dopant; **6** is a HBL comprising BCP; **7** is an ETL comprising Alq₃; **8** is an EIL comprising LiF and **9** is an Al layer cathode.

Some examples of the present invention are reported hereinafter, whose purpose is merely illustrative but not limitative of the scope of the invention itself.

### Example 1

### Synthesis of 3-Bromo-SBF

### Step 1: Synthesis of 3-Bromofluorenone

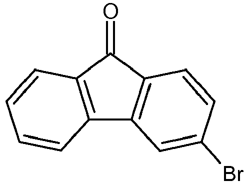

In a three ways flask 60 ml of water were added to 8.9 ml of hydrochloric acid (HCl, 37 % w/w, 2.1 molar equivalents) and the medium was cooled to 0°C. NaNO₂ (1.5 molar equivalents), dissolved in 50 ml of water, was added dropwise at 0°C. At the end of the addition, 4-amino-2-bromobenzophenone (one equivalent, 15.0 g, 51.6 mmole) solubilised in a mixture of acetone/water (400/230 ml), was added carefully. After 30 minutes at room temperature, the mixture was warmed up and kept at 60 °C for 3 hours.

After extraction with methylene chloride and evaporation of the organic phase, a brown solid was recovered (17.4 g) and a flash chromatography is realized. The pure compound was recovered after crystallization with hexane (4.2 g, 32 % yield).

### Step 2: 3-bromo-SBF

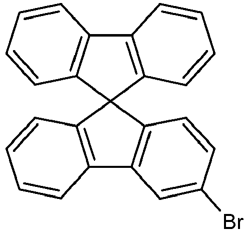

This compound was made in two steps from 3-bromofluorenone obtained in step 1. First, 2-bromobiphenyl (1.05 equivalents, 4.0 g, 16.5 mmol) was solubilised in 102 ml of anhydrous diethyl ether. This solution was cooled to -60°C and n-BuLi (1.16 eq.) is added dropwise. After 10 min at this temperature, a white precipitate appeared which was redissolved while the medium was warmed to room temperature. 3-Bromofluorenone was then added and the reaction mixture was kept at 45 °C for one night.

After addition of NH₄Cl (5% aq. 260 ml) and extraction with diethyl ether, 7.0 g of an alcohol was obtained. This solid was solubilised in 141 ml of acetic acid and hydrolyzed by the addition of 78 ml of HCl/dioxane (10 % mol, 20 eq.). After evaporation of the solvents, the solid was subjected to normal phase flash chromatography to afford 5.86 g of the target compound (94 % yield).

### Step 3: 3-N,N'-bis-(3-methylphenyl)amino -SBF

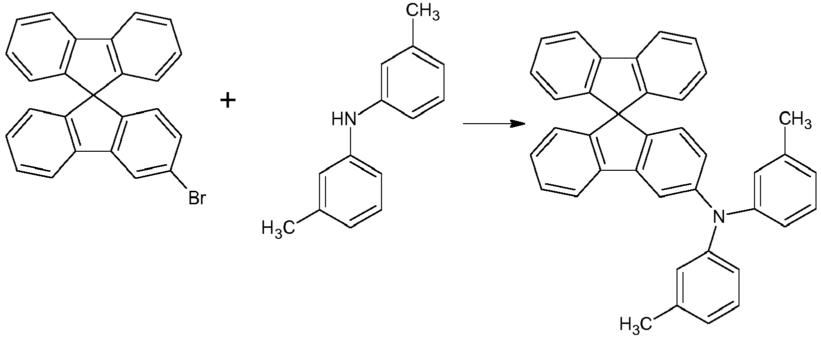

In a 500 ml flask, 320 ml of toluene were degassed during 2 hours. 3-Bromo SBF (9.9 g, 0.025 mol), sodium tert. butylate (tBuONa, 4.92 g, 0.05 mol) and 3,3'-dimethyldiphenylamine (5g, 0.025 mol) were added and after 30 min, a mixture of Bis-dibenzylideneacetone palladium (Pd(dba)₂) and Tris-tert.-butylphosphine (P(tBu)₃) previously prepared was slowly added.

The solution was warmed to 110 °C for one hour and then filtrated on a celite pad. After evaporation of the solvent, the solid recovered was recrystallised in ethanol to afford 5.5 g of a white solid (42 % yield).

## Claims

1. Compounds of general formula (3) 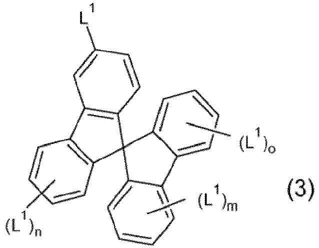
wherein n, m and o are zero,
each of the phenyl rings may carry no ligands other than L¹ or may be substituted by ligands other than L¹,
L¹ is represented by 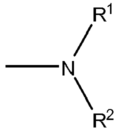 wherein R¹ and R² independently from each other are unsubstituted or substituted phenyl or naphthyl.

2. Compounds in accordance with claim 1 represented by the general formula 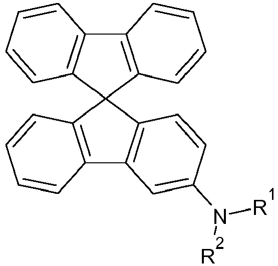 wherein R¹ and R² are as defined in claim 1.

3. Use of the compounds in accordance with at least one of claims 1 to 2 in an organic light emitting device.

4. The use of claim 3 wherein the light emitting device is an organic light emitting diode.

5. Use of the compounds according to at least one of claims 1 to 2 as host in an emissive layer in an organic light emitting device.

6. An organic light emitting device (OLED) comprising an emissive layer (EML), said emissive layer comprising at least one compound according to at least one of claims 1 to 2 with an emitting material.

## Patentansprüche

1. Verbindungen der allgemeinen Formel (3) 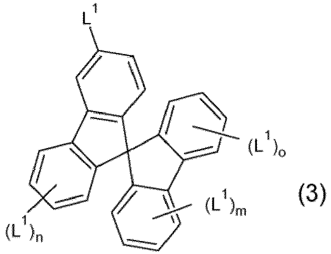
wobei n, m und o für null stehen,
jeder der Phenylringe keine Liganden außer L¹ tragen kann oder außer L¹ durch Liganden substituiert sein kann,
L¹ durch 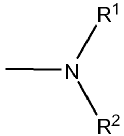 wiedergegeben wird, wobei R¹ und R² unabhängig voneinander für unsubstituiertes oder substituiertes Phenyl oder Naphthyl stehen.

2. Verbindungen nach Anspruch 1, die durch die allgemeine Formel 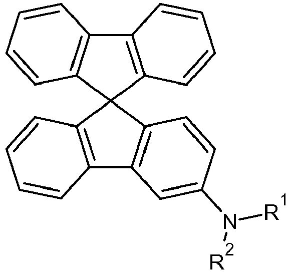 wiedergegeben werden, wobei R¹ und R² wie in Anspruch 1 definiert sind.

3. Verwendung der Verbindungen nach mindestens einem der Ansprüche 1 bis 2 in einer organischen lichtemittierenden Vorrichtung.

4. Verwendung nach Anspruch 3, wobei es sich bei der lichtemittierenden Vorrichtung um eine organische Leuchtdiode handelt.

5. Verwendung der Verbindungen nach mindestens einem der Ansprüche 1 bis 2 als Wirt in einer Emissions schicht in einer organischen lichtemittierenden Vorrichtung.

6. Organische lichtemittierende Vorrichtung (OLED) mit einer Emissionsschicht (EML), wobei die Emissionsschicht mindestens eine Verbindung nach mindestens einem der Ansprüche 1 bis 2 mit einem emittierenden Material umfasst.

## Revendications

1. Composés de formule générale (3) 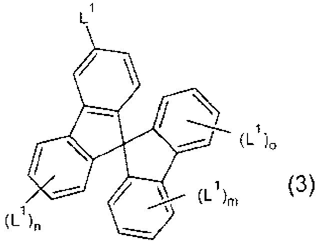
dans laquelle n, m et o sont zéro,
chacun des cycles phényle peut ne comporter aucun ligand autre que L¹ ou peut être substitué par des ligands autres que L¹,
L¹ est représenté par 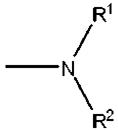 dans lequel R¹ et R², indépendamment l'un de l'autre, sont phényle ou naphtyle non substitué ou substitué.

2. Composés selon la revendication 1, représentés par la formule générale 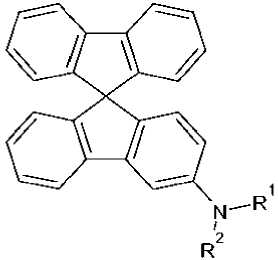 dans laquelle R¹ et R² sont tels que définis dans la revendication 1.

3. Utilisation des composés selon au moins une des revendications 1 à 2 dans un dispositif électroluminescent organique.

4. Utilisation selon la revendication 3, dans laquelle le dispositif électroluminescent est une diode électroluminescente organique.

5. Utilisation des composés selon au moins une des revendications 1 à 2 en tant qu'hôte dans une couche émissive dans un dispositif électroluminescent organique.

6. Dispositif électroluminescent organique (OLED) comprenant une couche émissive (EML), ladite couche émissive comprenant au moins un composé selon au moins une des revendications 1 à 2 avec un matériau émetteur.
